Europäisches Patentamt

European Patent Office

Office européen des brevets

(11) Numéro de publication: **0 214 168 B1**

(12) **FASCICULE DE BREVET EUROPEEN**

(45) Date de publication du fascicule du brevet: **19.09.90**

(51) Int. Cl.⁵: **A 61 F 13/15**

(21) Numéro de dépôt: **86901087.6**

(22) Date de dépôt: **06.02.86**

(86) Numéro de dépôt international:
**PCT/FR86/00033**

(87) Numéro de publication internationale:
**WO 86/05089 12.09.86 Gazette 86/20**

(54) **STRUCTURE ABSORBANTE POUR ARTICLE A USAGE UNIQUE.**

(30) Priorité: **01.03.85 FR 8503016**

(43) Date de publication de la demande:
**18.03.87 Bulletin 87/12**

(45) Mention de la délivrance du brevet:
**19.09.90 Bulletin 90/38**

(84) Etats contractants désignés:
**AT BE CH DE FR GB IT LI LU NL SE**

(56) Documents cités:
**DE-A-2 948 044**
**DE-A-3 002 136**
**GB-A-2 004 201**
**GB-A-2 014 046**
**US-A-3 903 889**

(73) Titulaire: **KAYSERSBERG SA**
**Route de Lapoutroie**
**F-68240 Kaysersberg (FR)**

(72) Inventeur: **PIERRE, Michel**
**2, passage de l'Hôtel de Ville**
**F-68100 Mulhouse (FR)**
Inventeur: **RUPPEL, Rémy**
**7, rue de Sorbiers**
**F-68000 Horbourg (FR)**
Inventeur: **BRELLMANN, Jean**
**5, petite rue des Tanneurs**
**F-68000 Colmar (FR)**

(74) Mandataire: **David, Daniel**
**KAYSERSBERG 54, avenue Hoche**
**F-75008 Paris (FR)**

Courier Press, Leamington Spa, England.

# EP 0 214 168 B1

**Description**

L'invention se rapporte à une structure absorbante pour article à usage unique tel qu'une couche ou un change pour bébé, une serviette périodique ou tout autre article destiné à absorber et retinir les sécrétions du corps et à être jeté après usage.

Ce type d'article comporte en général une masse absorbant les liquides disposée entre un voile perméable côté corps et une feuille imperméable en polyéthylène côté exterieur.

La masse absorbante est le plus souvent constituée principalement d'une ou de plusieurs nappes de fibres cellulosiques obtenues par défibrage à sec de feuilles ou de rouleaux de pâte à papier.

Afin d'améliorer le pouvour absorbant du matelas et d'en limiter le volume il est connu d'y incorporer des additifs à rétention améliorée appelés également superabsorbants. Ces additifs, qui si présentent à sec sous forme de fibres, de film ou plus généralement de particules de granules ou pailletts sont des composés qui gonflent en présence de liquide à absorber, mais ils ne se solubilisent pas. Le liquide est alors absorbé de façon quasi irréversible, au moins dans les conditions usuelles d'emploi, et forme avec le composé un gel dont le volume peut être de plusieurs dizaines de fois supérieur à celui de l'additif sec.

Selon la plupart des techniques connues, les particules superabsorbantes sont fixées à la surface du matelas ou incorporées dans la masse. Les termes "fixées" ou "incorporées" signifiant qu'il existe une liaison rigide entre la particule et le matelas obtenue par la présence d'un produit additif responsable de l'adhésion.

On connait par exemple par le brevet FR 2402474 un procédé de fabrication d'une structure absorbante où l'additif à rétention améliorée est associé à un support, puis fixé grâce au dépôt d'un liant effectue par pulvérisation ou par transfert. Le support peut être un film synthétique constituant la face extérieure ou un matériau fibreux. Selon une des formes de réalisation du brevet la couche d'additif est recouverte d'un deuxième matelas fibreux avant la mise en place du voile perméable côté corps.

Selon la brevet FR 2446357, l'additif est contenu, sans y être fixé, entre deux nappes fibreuses liées entre elles suivant des lignes ou des points de cohésion. Ces nappes délimitent ainsi un certain nombre de réceptacles dans lesquels peut se développer librement le gel.

Selon une autre technique connue une couche pour bébé est réalisée en disposant successivement sur une feuille de tissue-ouate, un matelas de mousse de cellulose, puis une couche de superabsorbant en une bande étroite, centrale et une feuille perméable aux liquides de la dimension de la bande de superabsorbant. Les bords latéraux de la feuille de tissue-ouate et du matelas non recouverts de superabsorbant sont ensuite repliés l'une sur l'autre sur la feuille perméable. On obtient ainsi une couche à section en forme d'escargot, à triple épaisseur de mousse de cellulose emprisonnant le superabsorbant.

Ces différents procédés permettent la réalisation de produits présentant une bonne capacité d'absorption de liquides.

Cependant il est apparu que, pou une certain pourcentage des articles comportant une des structures présentées ci-dessus, le gel devenait visible après un usage normal. Cela même lorsque l'additif était incorporé dans la masse, en profondeur, de façon à laisser côté corps, immédiatement sous la feuille perméable, une nappe de mousse de cellulose exempte ou pauvre en additif permettant la diffusion du liquide ves les couches proches de la feuille imperméable extérieure.

Le fait que le gel devienne visible peut être dû à la perte de cohésion de cette nappe, favorisée par les mouvements du bébé. Cela peut être dû aussi à la migration du gel à travers cette nappe. Lorsqu'on a introduit une feuille perméable entre l'additif et la nappe de fluff supérieure on a pu constater une amélioration; mais celle-ci n'était pas significative, une migration se produisait également à travers les mailles ou les perforations de la feuille, ou bien le gel contournait celle-ci. Cette migration n'est pas souhaitable car elle favorise le bouloche de la couche nuisant au confort de l'utilisateur. Par ailleurs une mauvaise répartition entraîne un effet de barrage par le gel formé en certains endroits augementant les risques de fuite. Dans des conditions extrèmes d'utilisation le gel peut traverser le voile perméable côté corps et se déposer sur la peau du bébé. La consistance visqueuse de ce gel, bien qu'inoffensif, n'est certainement pas très engageante et augmente le sentiment d'inconfort.

Selon le brevet US 3903889 on dispose une ou plusieurs feuilles de tissue-ouate entre la masse absorbante chargée de particules superabsorbantes et le voile perméable supérieur. Une couche de colle lie la première feuille à la masse absorbante. A l'usage, une telle structure ne donne pas satisfaction car les feules de tissue-ouate ne sont pas résistantes à l'humidité et ne s'opposent pas suffisamment à la pression du gel sous-jacent, par ailleurs l'association de plusieurs feuilles dans le but d'améliorer la tenue à l'humidité présente l'inconvénient de nuire à la diffusion rapide des liquides et augmente les risques de fuite.

L'invention a donc pour but de rémedier à ces inconvénients; elle propose une structure absorbante, présentant des qualités améliorées de tenue et de résistance, pour laquelle le risque de voir remonter le gel sous la surface de la feuille perméable côté corps est fortement réduit.

Conformément à l'invention, une structure absorbante pour article à usage unique, comportant successivement au moins une feuille perméable côté corps, une nappe composée exclusivement de fibres cellulosiques obtenue par défibrage à sec de pâte à papier, une couche d'adhésif, une couche absorbante contenant des particules d'additif à rétention améliorée et une feuille imperméable extérieure est caractérisée en cen que ladite couche d'adhésif assure la liaison d'une feuille de renfort non-tissé résistant

2

humide avec ladite couche absorbante, et en ce que ladite nappe est interposée entre ladite feuille de renfort et la feuille perméable.

Un non-tissé est un produit manufacturé de surface étendue par rapport à son épaisseur, constitué de fibres individuelles orientées suivant une direction ou au hasard liées par un procédé physique ou chimique, à l'exclusion du papier et de produits obtenus par tissage, tricotage, feutrage etc... Les fibres peuvent être d'origine naturelle ou chimique et de largeur finie ou indéfinie, elles peuvent être formées in situ. Dans son application à l'invention un non-tissé se distingue du papier par le fait qu'il ne se déchire pas à l'état humide sous la pression du gel qui restera ainsi masqué.

Ainsi, la résistance sens travers, humide, d'une éprouvette longue de 200 mm et large de 50 mm est inférieure à 150 cN pour un papier multiplis de grammage 50 g/m$^2$ alors que pour un non-tissé de grammage 20 g/m$^2$ elle doit être supérieure à 200 cN.

Selon une réalisation préférentialle le collage est effectué par dépot de colle thermofusible, technique nommée en anglais "spray hotmelt".

Sans nuire à la capacité d'absorption de la structure et tout en permettant aux particules d'additif de se développer sans obstacle, le collage assure une grande stabilité structurale; les tests réalisés ont montré une amélioration surprenante au regard du taux de gel visible après usage. Ce taux est défini comme la rapport entre le nombre de changes où le gel était visible après usage et le nombre de changes observés.

D'autres caractéristiques et avantages sont développés dans la description détaillée qui suit, se rapportant à quatre modes de réalisation, non limitatifs, de l'invention et où:

la figure 1 représente en coupe transversale une première forme de réalisation de la structure absorbante selon l'invention

la figure 2 représente un deuxième mode de réalisation en coupe transversale

la figure 3 représente un troisième mode de réalisation en coupe transversale

la figure 4 représente un quatrième mode de réalisation en coupe transversale

la figure 5 donne un schéma de principe d'un procédé de fabrication de la structure selon la figure 1

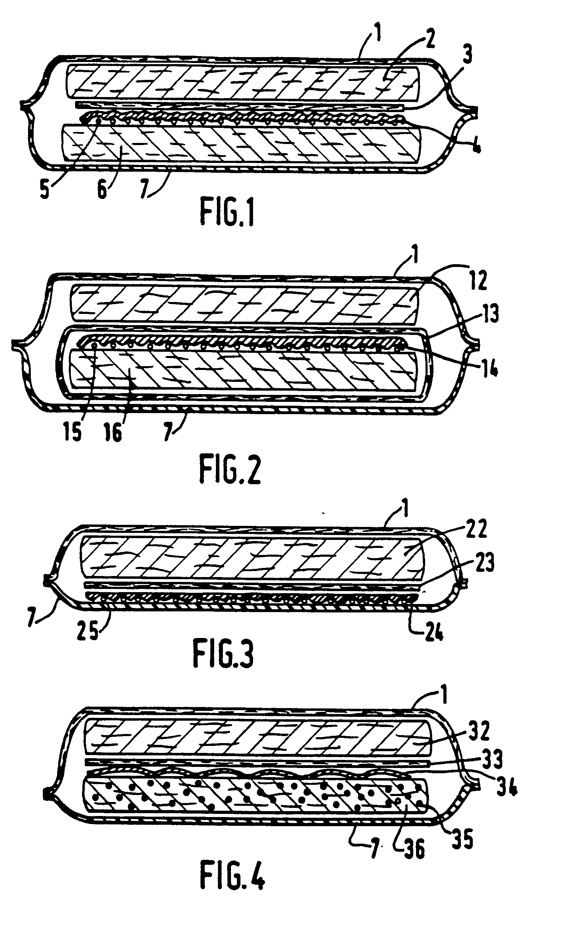

La figure 1 représente schématiquement la coupe transversale d'un article à usage que l'on considèrera par la suite comme étant un change pour bébé, pour fixer les idées; les éléments sont disposés conformément à un premier mode de réalisation de l'invention.

Ce change est recouvert sur sa face supérieure, celle qui vient au contact de la peau du bébé, d'une feuille perméable (1) aux liquides corporels. Cette feuille peut être une feuille imperméable percée d'une multitude de trous ou bien un non-tissé tel que celui vendu sous la marque HOLNEST qui est un voile de fibres de polypropylène thermoliées. D'autres types de feuilles perméables sont également utilisables.

Le matelas absorbant, proprement dit, est constitué d'une première nappe de fibres cellulosiques 2, mousse de cellulose par exemple comprenant ou non des fibres synthétiques.

Sous la nappe 2 est disposée une feuille de renfort intérieure 3 perméable aux liquides, de même largeur que la nappe 2.

Cette feuille de renfort peut être de même nature que la feuille extérieure 1 ou de nature différente. Mais elle doit être suffisamment résistant pour que sa cohésion soit maintenue après mouillage; cela exclut donc les tissues-ouates. De préférence cette feuille sera de plus faible grammage que la feuille perméable extérieure.

Il peut s'agir par exemple d'une grille réalisée selon l'enseignement du brevet FR 2195555 et connue sous la marque SCRINYL, grille en polypropylène renforcée par des filaments de polyamide, ou bien, d'un voile de fibres thermoplastiques liées, comme le HOLNEST. Le non-tissé 3 est maintenu sur une deuxième nappe de fibres 6, de même nature que la première, au moyen d'un liant 4. Le pliant peut être du type de ceux couramment utilisés dans des applications semblables, par exemple un polyvinylalcool dont le dépôt est obtenu par pulvérisation d'une dispersion diluée du liant. Ce type de liant, en dilution, présente cependant l'inconvénient d'humidifier le support pendant la fabrication ce qui en grève le coût.

Pour ces raisons un liant du type "Spray Hotmelt" est préferé. Il s'agit d'une technique de pulvérisation à chaud de filaments de colle thermofusible, par exemple celle vendue sous la marque BELIX 74101 par BERICOL.

Le liant a également pour fonction de maintenir sur le matelas de fibres 6, les particules de superabsorbant 5 disposées en une couche.

Ces additifs peuvent être ioniques comme l'AKUCELL de ENKA qui est un dérivé cellulosique du type CMC-réticulée, le SANWET de SANYO, le SGP de HENKEL où le A—100 de GRAIN PROCESSING CORP. qui sont des dérivés d'amidon, le FAVOR de STOCKHAUSEN, l'AQUALIC de NIPPON SHOKUBAI, l'AQUAQEEP de SEITETSU qui sont des dérivés de synthèse. Ces additifs peuvent être aussi non ioniques du type hydroxyéthylcellulose ou polyéthylène oxyde réticulé et sont alors peu sensibles à la salinité des liquides.

Cette structure absorbante est recouverte sur sa face extérieure opposée à la face 1, d'une feuille 7 imperméable aux liquides en polyéthylène. Les feuilles 1 et 7 sont liées par leurs bords de façon à enfermer le matelas absorbant.

La figure 2 représente un deuxième mode de réalisation de l'invention.

Dans cette variante le feuille de renfort perméable 13 s'étend autour de la deuxième nappe 16 de manière à former une enveloppe de la nappe 16 et de l'additif 15. De cette façon on garantit encore un meilleur maintien du gel. La colle 14 maintient à la fois la feuille de renfort 13 et l'additif 15 sur la nappe 16.

La figure 3 représente un troisième mode de réalisation où la nappe de fibres inférieure a été supprimée. La deuxième nappe absorbante est ainsi constituée uniquement d'additif 25 à rétention améliorée, la colle 24 maintient la feuille de renfort 23 contre la feuille imperméable 7.

La figure 4 représente un quatrième mode de réalisation où les particules d'additif 35 sont incorporées dans la masse de la deuxième nappe 36, le non-tissé 33 est fixé par le liant 34 à cette nappe 36.

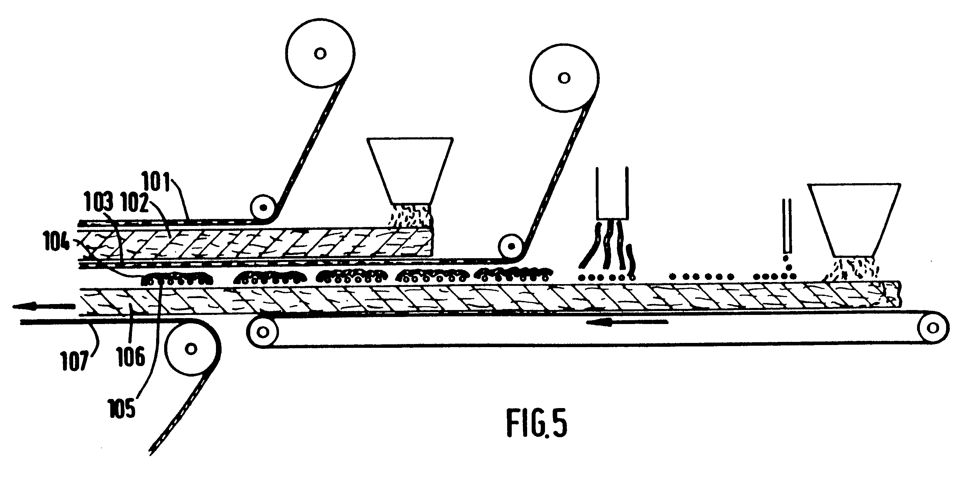

La figure 5 donne un schéma de principe du procédé de fabrication de la structure selon le premier mode de réalisation.

La mousse de cellulose est déposée en une nappe 106 d'épaisseur uniforme sur une brande transporteuse, une buse dépose à intervalles réguliers une couche de particules de superabsorbant 105 en quantité déterminée; elle passe sous un pistolet de pulvérisation à chaud de filaments de colle thermofusible 104 (ce pistolet comprend une buse par laquelle la colle fondue est injectée dans un courant d'air chaud; celui-ci éclate le jet de colle en filaments). Puis une feuille de non-tissé 103 est déposée sur la colle. Sur cette feuille on dépose une nappe 102 de mousse de cellulose d'épaisseur uniforme. Cet ensemble est ensuite transféré sur une feuille 107 de polyéthylène et une feuille perméable supérieure 101 vient compléter la structure qui est ensuite calendrée, scellée et découpée pour former l'article voulu.

Des essai comparatifs ont été effectués en pouponnière pour tester des changes complete fabriqués conformément au premier mode de réalisation de l'invention.

Il s'agissait de changes comportant deux nappes superposées de mousse de cellulose de grammage 450 g/m² chacune, l'additif éait du SANWET à raison de 1,5 à 2 g par change. La feuille perméable supérieure était du HOLNEST de grammage 19 g/m², la feuille imperméable du polyéthylène. On a constitué 6 lots de deux cents changes chacun; les lots étaient respectivement:

1) sans feuille intérieure

2) avec une grille de renfort intérieure en SCRINYL (9 g/m²) non collée à la nappe inférieure

3) avec un grille de renfort intérieure en SCRINYL (9 g/m²) collée à la nappe inférieure avec une colle polyvinylalcool diluée à 5%, à raison de 2 g/m² de colle

4) avec un grille de renfort en SCRINYL (9 g/m²) collée "hotmelt" à la nappe inférieure a raison de 7 g/m² de colle BELIX 74101

5) avec un non-tissé HOLNEST (11 g/m²) non collé

6) avec un non-tissé HOLNEST (11 g/m²) collé "hotmelt" à la nappe inférieure a raison de 7 g/m² de colle BELIX 74101

Après usage on a recensé le nombre de changes dont le gel était visible à travers le non-tissé extérieur; ce nombre traduit en pourcentage, on obtient un taux de gel visible par lot. On a recensé également le nombre de changes dont la nappe était détériorée.

Les résultats sont les suivants:

|  | taux de gel visible % | nappe déteriorée % |
|---|---|---|
| 1) | >45 | 35 |
| 2) | 25 | 11 |
| 3) | 15 | 5 |
| 4) | 4 | 2 |
| 5) | 9 | 2 |
| 6) | 0 | 0 |

Ces résultats montrent:

Une forte migratin du gel en l'absence de feuille de renfort intérieure, (lot 1))

Quand on utilise un grille SCRINYL on réduit fortement le taux de gel visible par collage (lots 3) et 4)) — ce taux est encore amélioré quand on utilise une colle thermofusible (lot 4)) plutôt qu'une colle diluée pulverisée (lot 3)). Cela s'explique par le fait que l'on doit limiter la quantité de cette dernière pour éviter de trop mouiller la nappe.

On obtient un résultat excellent quand on utilise un non-tissé léger, HOLNEST, lié à la nappe inférieure au moyen d'une colle thermofusible (lot 6)). Le meilleur résultat par rapport à la grille SCRINYL s'explique sans doute par le fait que le non-tissé est de maille plus serrée que la grille pour un grammage équivalent.

On peut réaliser grâce à une structure conforme à l'invention (lots 3), 4), 6)) des changes minces nécessitant moins de maitère et dont les propriétés sont satisfaisantes au regard du confinement du gel et du maintien de la nappe. Ces résultats sont notamment à rapprocher de ceu concernat les changes à section en forme d'escargot mentionnés plus haut, plus volumineux où pour une nappe de 350 g/m² repliée donnant un matelas de 700 g/m² entre le non-tissé côté corps et l'additif, on obtenait un taux de gel visible de 20% sans non-tissé intermédiare et, 8% et 4% avec un non-tissé de renfort intérieur en SCRINYL respectivement HOLNEST non collé.

4

**Revendications**

1. Structure absorbante pour article à usage unique, comportant successivement, au moins une feuille perméable (1) côté corps, une nappe (2, 12, 22, 32) composée exclusivement de fibres cellulosiques et obtenue par défibrage à sec de pâte à papier, une couche d'adhésif, une couche absorbante (5—6, 15—16, 25, 35—36) contenant des particules d'additif à rétention améliorée et une feuille imperméable extérieure (7), caractérisée en ce que ladite couche d'adhésif assure la liaison d'une feuille de renfort en non-tissé (3, 13, 23, 33) résistant humide avec ladite couche absorbante, et en ce que ladite nappe (2, 12, 22, 32) est interposée entre ladite feuille de renfort et la feuille perméable.

2. Structure absorbante selon la revendication 1 caractérisée en ce que la couche absorbante est constituée d'une nappe de fibres (6), l'additif (5) étant déposé en surface sur toute ou partie de la face de ladite nappe adjacente à la feuille de renfort intérieure (3).

3. Structure absorbante selon la revendication 2, caractérisée en ce que la feuille de renfort 13 enveloppe la couche absorbante (15, 16).

4. Structure absorbante selon la revendication 1, caractérisée en ce que la couche absorbante est constituée essentiellement d'additif à rétention améliorée (25), la colle (24) maintenant la feuille de renfort (23) contre la feuille imperméable (7).

5. Structure absorbante selon la revendication 1, caractérisée en ce que la couche absorbante est constituée d'une nappe de fibres (36) incorporant des particules d'additif à rétention améliorée (35).

6. Structure selon l'un des revendications précédentes caractérisée en ce que la colle (4, 14, 24, 34) est une colle thermofusible.

7. Structure absorbante selon l'une des revendications précédentes caractérisée en ce que la feuille de renfort est une grille.

8. Structure absorbante selon l'une des revendications précédentes caractérisée en ce que la feuille de renfort est un voile de fibres thermoplastiques liées.

**Patentansprüche**

1. Absorbierende Struktur für Artikel für einmalige Benutzung, nacheinander enthaltend, zumindest eine durchlässige Folie zur Seite des Körpers, ei Vlies (2, 12, 22, 32), welches ausschließlich aus Cellulosefasern zusammengesetzt ist und durch Entfaserung und Trocknen von Papiermasse erhalten ist, eine klebende Schicht, eine absorbierende Schicht (5—6, 15—16, 25, 35—36), die Zusatzstoffteilchen zum verbesserten Zurückhalten und eine undurchlässige äußere Folie (7) enthält, dadurch gekennzeichnet, daß die klebende Schicht die Verbindung eine Verstärkungsfolie aus feuchtigkeitsbeständigem Nonwoven (3, 13, 23, 33) mit der absorbierenden Schicht sichert, und daß das Vlies (2, 12, 22, 32) zwischen die Verstärkungsfolie und die durchlässige Folie gelegt ist.

2. Absorbierende Struktur gemäß Anspruch 1, dadurch gekennzeichnet, daß die absorbierende Schicht aus einer Schicht von Fasern (6) besteht, wobei der Zusatzstoff (5) auf der Oberfläche auf der gesamten oder einem Teil der Seite der Schicht benachbart zur inneren Verstärkungsfolie (3) angeordnet ist.

3. Absorbierende Struktur gemäß Anspruch 2, dadurch gekennzeichnet, daß die Verstärkungsfolie (13) die absorbierende Schicht (15, 16) umhüllt.

4. Absorbierende Struktur gemäß Anspruch 1, dadurch gekennzeichnet, daß die absorbierende Schicht im wesentlichen aus einem Zusatzstoff mit verbesserter Zurückhaltung (25) besteht, wobei der Klebstoff (24) die Verstärkungsfolie (23) an der undurchlässigen Folie (7) festhält.

5. Absorbierende Struktur gemäß Anspruch 1, dadurch gekennzeichnet, daß die absorbierende Schicht aus einer Schicht von Fasern (36) besteht, welche die Zusatzstoffteilchen mit verbesserter Zurückhaltung (35) aufnimmt.

6. Absorbierende Struktur gemäß einem der vorstehenden Ansprüche, dadurch gekennzeichnet, daß der Klebstoff (4, 14, 24, 34) ein wärmeschmelzbarer Klebstoff ist.

7. Absorbierende Struktur gemäß einem der vorstehenden Ansprüche, dadurch gekennzeichnet, daß die Verstärkungsfolie ein Gitter ist.

8. Absorbierende Struktur gemäß einem der vorstehenden Ansprüche, dadurch gekennzeichnet, daß die Verstärkungsfolie ein Flor miteinander verbundener thermoplastischer Fasern ist.

**Claims**

1. An absorbant structure for a disposable article comprising in succession at least one permeable sheet (1) on the body side, a layer (12, 12, 22, 32,) consisting of cellulose fibres only and obtained by the dry teasing of paper paste, a layer of adhesive, and absorbent layer (5—6, 15—16, 25, 35—36) containing improved retention additive particles and an outer impermeable sheet (7), characterised in that the said layer of adhesive provides a bond between a non-woven reinforcing sheet (3, 13, 23, 33), which has wet strength, with the said absorbent layer, and in that the said layer (2, 12, 22, 32) is placed between the said reinforcing sheet and the permeable sheet.

2. An absorbent structure according to claim 1, characterised in that the absorbent layer consists of a layer of fibres (6), additive (5) being deposited on the surface of all the face of the said sheet adjacent to the inner reinforcing sheet (3).

3. An absorbent structure according to claim 2, characterised in that reinforcing sheet 13 encloses the absorbent layer (15, 16).

4. An absorbent structure according to claim 1, characterised in that the absorbent layer consists essentially of improved retention additive (25) adhesive (24) holding reinforcing sheet (23) against the impermeable sheet (7).

5. An absorbent structure according to claim 1, characterised in that the absorbent layer consists of a layer of fibres (36) incorporating particles of improved retention additives (35).

6. A structure according to one of the foregoing claims characterised in that the adhesive (4, 14, 24, 34) is a thermally activated adhesive.

7. An absorbent structure according to one of the foregoing claims characterised in that the reinforcing sheet is a grid.

8. An absorbent structure according to one of the foregoing claims characterised in that the reinforcing sheet is a sheet of bonded thermoplastic fibres.

FIG.1

FIG.2

FIG.3

FIG.4

FIG.5